# FASCICULE DE BREVET EUROPEEN

(11) **EP 3 826 655 B1**
(45) Date de publication et mention de la délivrance du brevet: **19.10.2022**
(21) Numéro de dépôt: 19734004.5
(22) Date de dépôt: 19.06.2019
(51) Int. Cl.: A61K 35/744, A61P 1/12, A61P 37/06, A61P 1/00, A23L 33/135, A23C 9/123, C12N 1/20

(54) **SOUCHE DE STREPTOCOCCUS THERMOPHILIUS CNRZ160 POUR LE TRAITEMENT ET LA PRÉVENTION DE L'INFLAMMATION INTESTINALE ET DES DÉSORDRES ASSOCIÉS, CHEZ UN INDIVIDU**
STREPTOCOCCUS THERMOPHILUS CNRZ160-STAMM ZUR BEHANDLUNG UND PRÄVENTION VON DARMENTZÜNDUNG UND EINHERGEHENDEN ERKRANKUNGEN BEI EINER PERSON
STREPTOCOCCUS THERMOPHILUS CNRZ160 STRAIN FOR THE TREATMENT AND PREVENTION OF INTESTINAL INFLAMMATION AND ASSOCIATED DISORDERS IN AN INDIVIDUAL

(30) Priorité: 26.07.2018 FR 1856950
(43) Date de publication de la demande: 02.06.2021
(73) Titulaire: Institut national de recherche pour l'agriculture, l'alimentation et l'environnement, 75007 Paris (FR); Université de Lorraine, 54000 Nancy (FR); Université Clermont Auvergne, 63000 Clermont-Ferrand (FR)
(72) Inventeur: DARDEVET, Dominique, 63170 Perignat lès Sarlieve (FR); AUZELOUX, Isabelle, 63000 Clermont Ferrand (FR); JARZAGUET, Marianne, 63410 Loubeyrat (FR); DAVID, Jérémie, 63110 Beaumont (FR); CHATEL, Jean-Marc, 92190 Meudon (FR); DARY-MOUROT, Annie, 54200 Bicqueley (FR); CHERBUY, Claire, 91940 Les Ulis (FR); LANGELLA, Philippe, 78140 Vélizy-Villacoublay (FR)
(74) Mandataire: Nony
(86) Numéro de dépôt international: PCT/EP2019/066178
(87) Numéro de publication internationale: WO 2020/020540

(56) Documents cités:
- WO-A2-2017/137920
- FR-A1- 3 046 934
- MARTIN R ET AL: "The commensal bacterium faecalibacterium prausnitzii is protective in DNBS-induced chronic moderate and severe colitis models", HHS PUBLIC ACCESS AUTHOR MANUSCRIPT, LIPPINCOTT WILLIAMS & WILKINS, US, vol. 20, no. 3, 1 janvier 2014 (2014-01-01), pages 417-430, XP009191583, ISSN: 1078-0998, DOI: 10.1097/01.MIB.0000440815.76627.64 cité dans la demande
- SCHELLACK N: "Prebiotics, probiotics and synbiotics: An update", SA PHARMACEUTICAL JOURNAL 2018 MEDPHARM PUBLICATIONS ZAF, vol. 85, no. 5, 2018, pages 22-28, XP055593789, ISSN: 1015-1362
- CREMON CESARE ET AL: "Pre- and probiotic overview", CURRENT OPINION IN PHARMACOLOGY, ELSEVIER SCIENCE PUBLISHERS, NL, vol. 43, 13 septembre 2018 (2018-09-13), pages 87-92, XP085555128, ISSN: 1471-4892, DOI: 10.1016/J.COPH.2018.08.010

## Description

### Domaine de l'invention

La présente invention concerne une souche de *Streptococcus thermophilus* de référence CNRZ160 pour son utilisation dans la prévention et/ou le traitement de l'inflammation intestinale, et de troubles provoqués par cette inflammation intestinale, chez un individu.

L'invention concerne plus particulièrement une souche de *Streptococcus thermophilus* de référence CNRZ160 pour son utilisation dans la prévention et/ou le traitement de la perte de masse maigre corporelle.

### Art antérieur

L'inflammation est un processus biologique naturel, qui constitue une partie normale de la réponse à des lésions ou des infections et contribue à la protection de l'organisme contre les agressions internes ou externes.

Cependant, un dysfonctionnement des mécanismes de l'inflammation, en particulier une inflammation persistante ou trop abondante, peut causer des maladies douloureuses et mettre en danger la vie du patient. De telles maladies comprennent, par exemple, des troubles cutanés, des troubles intestinaux, des troubles neurologiques, l'arthrite et des maladies auto-immunes. Plusieurs de ces maladies inflammatoires restent sans traitement ou sans traitement adéquat.

Une telle inflammation peut toucher tout ou partie de l'intestin. Ainsi, sont notamment distinguées, dans les cas où l'inflammation est localisée au niveau d'une région donnée, l'entérite (inflammation de l'intestin grêle), la duodénite (inflammation du duodénum), l'iléite (inflammation de l'iléon), la jéjunite (inflammation du jéjunum), la typhlite (inflammation du caecum), l'appendicite (inflammation de l'appendice) et la colite (inflammation du côlon). Lorsque plusieurs régions de l'intestin sont inflammées, on parle également d'entérocolite (inflammation de l'intestin grêle et du colon), de gastro-entérite (inflammation de l'estomac et de l'intestin grêle), de rectocolite hémorragique (aussi nommée colite ulcéreuse, maladie chronique inflammatoire des intestins qui touche plus spécifiquement le côlon et le rectum) et de maladie de Crohn (maladie chronique inflammatoire des intestins qui touche la totalité des intestins et peut s'étendre par exemple à la cavité buccale, au rectum ou au canal anal).

Par conséquent, l'étude et la recherche de nouvelles stratégies de traitement anti-inflammatoire constituent un sujet majeur en médecine et en recherche biomédicale.

Il existe ainsi un besoin constant de nouvelles substances, en particulier de probiotiques, ou de compositions, pour le traitement et/ou la prévention de l'inflammation intestinale.

Un individu souffrant ou ayant souffert d'une inflammation intestinale peut, en même temps ou après ladite inflammation, présenter des troubles pouvant être provoqués par cette inflammation, à savoir :
- une perte de poids ;
- une perte de masse maigre ;
- une perte musculaire ;
- une hypertrophie du côlon ; et/ou
- l'altération de la perméabilité intestinale.

Par conséquent, l'étude et la recherche de nouvelles stratégies de prévention et de traitement de la perte de poids, de la perte de masse maigre, de la perte musculaire, de l'hypertrophie du côlon et/ou d'une altération de la perméabilité intestinale constituent également un sujet majeur en médecine et en recherche biomédicale. Il existe ainsi également un besoin constant de nouvelles substances, en particulier de probiotiques, ou de compositions, pour le traitement et/ou la prévention de la perte de poids, de la perte de masse maigre, de la perte musculaire, de l'hypertrophie du côlon et/ou d'une altération de la perméabilité intestinale, en particulier chez un individu présentant une inflammation intestinale, et notamment pour le traitement et/ou la prévention de la perte musculaire.

### Résumé de l'invention

L'objectif de la présente invention est de décrire une nouvelle substance, en particulier un probiotique, et des compositions la comprenant, pour le traitement et/ou la prévention d'une affection intestinale inflammatoire et/ou d'un trouble provoqué par cette affection intestinale inflammatoire chez un individu selon les revendications.

Dans le contexte de la présente invention, les termes « prévenir » et « prévention » désignent la réduction à un degré moindre du risque ou de la probabilité d'occurrence d'un phénomène donné, c'est-à-dire, dans la présente invention, d'une affection intestinale inflammatoire et/ou d'un trouble provoqué par cette affection intestinale inflammatoire chez un individu.

Dans le contexte de la présente demande, les termes « traiter » et « traitement » associés à une affection intestinale inflammatoire et/ou un trouble provoqué par cette affection intestinale inflammatoire selon l'invention, désigne une diminution, voire une interruption de ladite affection intestinale inflammatoire et/ou dudit trouble provoqué par cette affection intestinale inflammatoire. Ainsi, par exemple, dans le cas du traitement de la perte de masse musculaire, ces termes désignent une diminution, voire une interruption, de la perte de masse musculaire, voire même une augmentation de la masse musculaire, cette augmentation étant matérialisée par une augmentation de la quantité de fibres musculaires au niveau du ou des muscle(s) concerné(s).

La présente invention est basée sur la découverte des propriétés de la souche de *Streptococcus thermophilus* de référence CNRZ160, déposée auprès de la Collection Nationale de Cultures de Micro-organismes (CNCM) (Institut Pasteur, 25-28, rue du Docteur Roux 75724 Paris Cedex 15) sous le numéro d'accession CNCM I-5334 le 4 juillet 2018, pour le traitement et la prévention, chez un individu, à la fois de l'inflammation intestinale mais également des troubles provoqués par cette affection intestinale inflammatoire.

Selon les résultats expérimentaux des inventeurs, une souche spécifique de *Streptococcus thermophilus* de référence CNRZ160 possède en effet la capacité inattendue de rétablir la perméabilité intestinale *in vivo* chez des individus présentant une inflammation intestinale.

De même, les résultats expérimentaux des inventeurs démontrent qu'une souche spécifique de *Streptococcus thermophilus* de référence CNRZ160 possède également la capacité inattendue de réduire *in vivo* la perte de poids, la perte de masse musculaire et la perte de masse maigre chez des individus présentant une inflammation intestinale.

Par ailleurs, d'autres résultats démontrent la capacité d'une souche spécifique de *Streptococcus thermophilus* de référence CNRZ160 à limiter, voire à annuler, l'hypertrophie du colon chez des individus présentant une inflammation intestinale.

De plus, les résultats expérimentaux des inventeurs démontrent également un retour à la normal de la synthèse protéique, aussi bien au niveau du colon que des muscles, après traitement avec une souche spécifique de *Streptococcus thermophilus* de référence CNRZ160.

Comme illustré dans les exemples, les inventeurs ont montré que les diverses propriétés de la souche CNRZ160 ne sont pas rencontrées en général chez *Streptococcus thermophilus.* Par exemple, la souche de *Streptococcus thermophilus* PB5MJ, déposée auprès de la CNCM sous le numéro d'accession CNCM I-5336 le 18 juillet 2018, ne possède pas cette propriété avantageuse.

Enfin, les présents résultats démontrent par ailleurs une absence de mortalité chez des individus présentant une inflammation intestinale et traités à l'aide d'une souche spécifique de *Streptococcus thermophilus* de référence CNRZ160. Cette propriété n'est également pas rencontrée en générale chez *Streptococcus thermophilus*, puisqu'à l'inverse, l'administration d'une souche de *Streptococcus thermophilus* PB5MJ entraine une augmentation très significative de la mortalité d'individus présentant une inflammation intestinale.

Ainsi, selon un premier objet, la présente invention concerne une souche bactérienne de l'espèce *Streptococcus thermophilus* déposée auprès de la CNCM sous le numéro d'accession CNCM I-5334, pour son utilisation dans la prévention et/ou le traitement d'une affection intestinale inflammatoire et/ou d'un trouble provoqué par cette affection intestinale inflammatoire, chez un individu.

Cette souche identifiée par les inventeurs est donc une souche probiotique qui peut être utilisée pour les applications indiquées ci-dessus.

Cette souche peut être mise en œuvre au sens de l'invention sous une forme vivante, semi-active, inactivée ou morte.

Au sens de l'invention, un microorganisme sous une forme semi-active est un microorganisme dont la capacité à proliférer est réduite, temporairement ou définitivement.

Ainsi, au sens de l'invention, un microorganisme « inactivé » est un microorganisme qui n'est plus capable, temporairement ou définitivement, de proliférer.

Au sens de l'invention, un microorganisme « mort » est un microorganisme qui n'est plus capable, définitivement, de proliférer.

Les microorganismes morts ou inactivés peuvent avoir les membranes cellulaires intactes ou rompues. Ainsi le terme « inactivé » désigne également les extraits et lysats de microorganismes obtenus comme détaillés ci-avant. L'obtention de microorganismes morts ou inactivés peut être effectuée par toute méthode connue de l'homme de l'art.

Un microorganisme probiotique inactivé convenant à l'invention peut être préparé par irradiation, inactivation thermique ou lyophilisation d'une préparation de microorganisme. Ces méthodes sont connues de l'homme de l'art.

Plus particulièrement, l'inactivation de microorganismes probiotiques par irradiation peut comprendre la mise en œuvre de rayons gamma, de rayons X ou une exposition aux UV. Le type de rayonnement, l'intensité, la dose et le temps d'exposition sont ajustés par l'homme de l'art selon la quantité et la nature des microorganismes probiotiques à inactiver.

L'inactivation par lyophilisation peut être effectuée par toute méthode connue dans le domaine. Avantageusement, des microorganismes probiotiques inactivés par lyophilisation peuvent être remis en culture.

Un microorganisme probiotique annexe selon l'invention peut être mis en œuvre sous forme entière, c'est-à-dire pour l'essentiel dans sa forme native, ou sous forme d'extraits ou de lysats comprenant des fractions et/ou des métabolites de ce microorganisme. Un tel lysat peut notamment être préparé comme indiqué ci-après.

Un lysat conforme à l'invention peut comprendre tout ou partie des éléments fractionnés et/ou des métabolites résultant de la lyse du microorganisme probiotique.

Un lysat au sens de l'invention désigne le produit obtenu à l'issue de la destruction ou dissolution de cellules biologiques par un phénomène de lyse cellulaire provoquant la libération des constituants biologiques intracellulaires naturellement contenus dans les cellules du microorganisme considéré.

Au sens de la présente invention, le terme « lysat » est utilisé indifféremment pour désigner l'intégralité du lysat obtenu par lyse du microorganisme concerné ou seulement une fraction de celui-ci.

Au sens de la présente invention, les termes « lysat entier » sont utilisés pour désigner plus précisément l'intégralité du lysat obtenu par lyse du microorganisme concerné.

Le lysat mis en œuvre est donc formé en tout ou partie des constituants biologiques intracellulaires et des constituants des parois et membranes cellulaires.

Selon un mode de réalisation, un lysat utilisé pour l'invention est l'intégralité du lysat obtenu par lyse du microorganisme concerné.

Cette lyse cellulaire peut être réalisée à l'aide de différentes technologies, telles que par exemple un choc thermique, par ultrasons, un choc osmotique, ou sous contrainte mécanique, telle que par centrifugation.

Un individu selon l'invention est de préférence un mammifère, y compris un mammifère non humain, et est, en particulier, un humain.

Selon un mode de réalisation particulier, ledit trouble provoqué par cette affection intestinale inflammatoire est choisi dans la liste constituée d'une perte de poids, d'une perte de masse maigre, d'une perte musculaire, d'une hypertrophie du côlon, d'une altération de la perméabilité intestinale et de l'une quelconque des combinaisons de ces troubles. Plus particulièrement, ledit trouble provoqué par cette affection intestinale inflammatoire est la perte de masse maigre et musculaire.

Ladite affection intestinale inflammatoire colique peut, en particulier, être une maladie inflammatoire chronique de l'intestin, et peut plus particulièrement être choisie dans le groupe constitué de la maladie de Crohn, la recto-colite hémorragique et la pochite, en particulier la maladie de Crohn et la recto-colite hémorragique.

Selon un autre mode de réalisation, la souche bactérienne pour son utilisation selon l'invention est comprise dans une composition comprenant un milieu physiologiquement acceptable, de préférence dans une composition orale.

Les termes « *milieu physiologiquement acceptable* » désignent un milieu qui est compatible avec l'organisme de l'individu auquel ladite composition doit être administrée. Il peut s'agir, par exemple, d'un solvant non toxique tel que l'eau. En particulier, ledit milieu est compatible avec une administration orale.

Une composition de l'invention est de préférence pour la voie orale.

Selon un mode de réalisation, la composition est adaptée pour l'administration d'une dose journalière représentant de 10⁷ à 10¹¹ unités formant des colonies (ufc), notamment en tant que médicament, de préférence sous la forme d'une dose journalière équivalente à 10⁹ ufc.

Une composition de l'invention pour une administration par voie orale peut être choisie dans le groupe constitué d'un produit alimentaire, d'une boisson, d'un produit pharmaceutique, d'un nutraceutique, d'un additif alimentaire, d'un supplément alimentaire et d'un produit laitier et est, préférentiellement, sous un produit laitier ou un supplément alimentaire.

### Légendes des figures

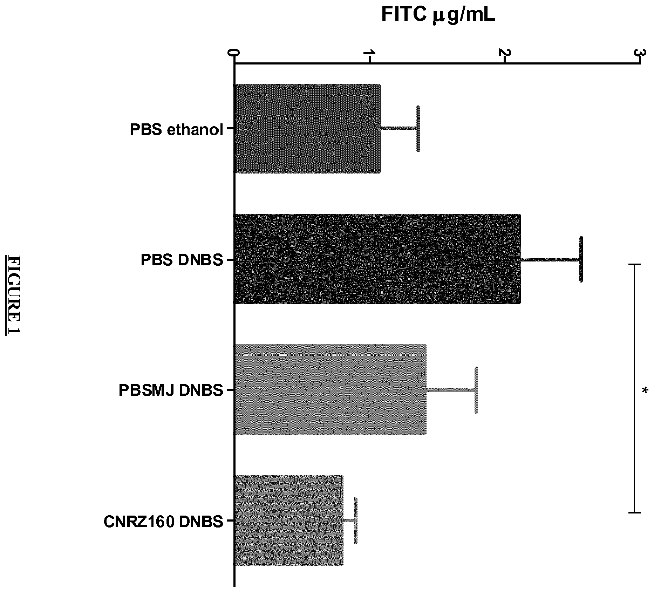

La Figure 1 illustre la perméabilité intestinale de différents groupes de souris (n=8/groupe) gavées ou non avec les souches PB5MJ ou CNRZ160, une inflammation bas grade ayant été induite chez ces animaux par l'administration de DNBS. La perméabilité intestinale est mesurée par un dosage de FITC dans le sang. Plus la concentration est élevée, plus la perméabilité intestinale est également élevée.

Les groupes de souris sont les suivants, de gauche à droite : PBS éthanol : groupe de souris traitées au PBS et à l'éthanol (contrôle non inflammé) ; PBS DNBS : groupe de souris traitées au DNBS et au PBS (contrôle inflammé) ; PB5MJ DNBS : groupe de souris traitées au DNBS et supplémentés en *Streptococcus thermophilus* PB5MJ ; CNRZ160 DNBS : animaux traités au DNBS et supplémentés en *Streptococcus thermophilus* CNRZ160.

Abscisse : groupes d'animaux testés.

Ordonnée : quantité de FITC dans le sang (µg/mL).

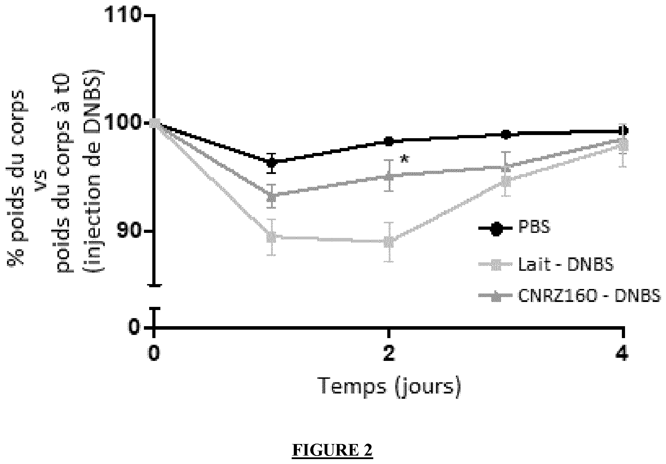

La Figure 2 illustre le pourcentage de poids perdu par différents groupes de souris par rapport à leur poids avant injection de DNBS déclenchant une inflammation intestinale aigüe.

Les groupes de souris sont les suivants : PBS : groupe de souris traitées au PBS (contrôle non enflammé) ; Lait - DNBS : groupe de souris traitées au DNBS et au Lait (contrôle enflammé) ; CNRZ160 - DNBS : animaux traités au DNBS et supplémentés en *Streptococcus thermophilus* CNRZ160.

Abscisse : nombre de jours post-injection de DNBS.

Ordonnée : Pourcentage de poids du corps restant par rapport au poids du corps à t0 (soit au jour de l'injection de DNBS).

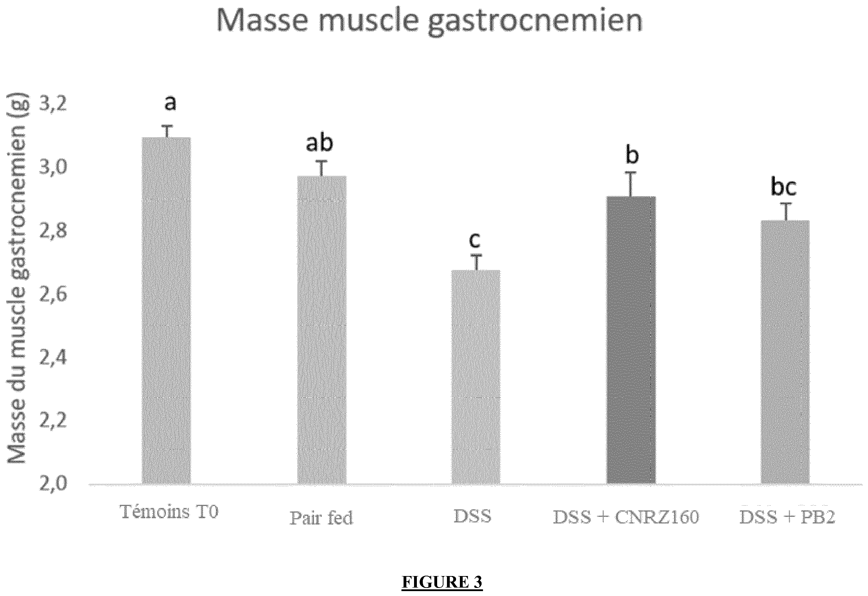

La Figure 3 illustre la masse du muscle gastrocnémien de différents groupes d'animaux : DSS = groupe d'animaux traités au Dextran Sulfate Sodium (DSS) pendant 28 jours ; Pair Fed = animaux pair-fed par rapport au groupe DSS ; DSS + CNRZ160 = animaux traités au DSS pendant 28 jours et supplémentés en *Streptococcus thermophilus* CNRZ160 ; DSS + PB2 = animaux traités au DSS pendant 28 jours et supplémentés en *Streptococcus thermophilus* PB5MJ, ainsi que d'un témoin correspondant à un groupe d'animaux euthanasiés au début de l'expérimentation pour mesurer le poids des muscles des rats avant traitement au DSS (Témoins T0). Chaque barre présentant une lettre différente d'une autre est significativement différente. Ainsi, les barres indiquant a, b ou c sont statistiquement différentes les unes des autres. La barre a est statistiquement différente des barres b, bc et c. La barre ab est statistiquement différente de la barre c. La barre c est statistiquement différente des barres a, b et ab. La barre b est statistiquement différente des barres a et c. La barre bc est statistiquement différente de la barre a.

Abscisse : groupes d'animaux testés.

Ordonnée : Masse du muscle gastrocnémien en gramme par rapport au groupe contrôle Témoins T0.

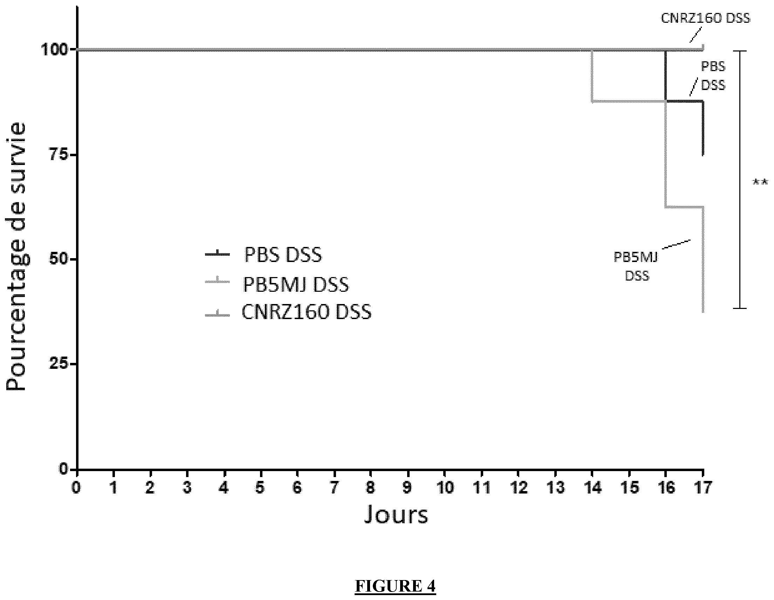

La Figure 4 illustre le pourcentage de survie de différents groupes de souris au bout de 17 jours post-administration de DSS.

Les groupes sont les suivants : PBS DSS : groupe de souris traitées au DSS et au PBS (contrôle enflammé) ; PB5MJ DSS : groupe de souris traitées au DSS et supplémentés en *Streptococcus thermophilus* PB5MJ ; CNRZ160 DSS : animaux traités au DSS et supplémentés en *Streptococcus thermophilus* CNRZ160.

Abscisse : nombre de jours post-injection de DSS.

Ordonnée : pourcentage de survie des souris traitées.

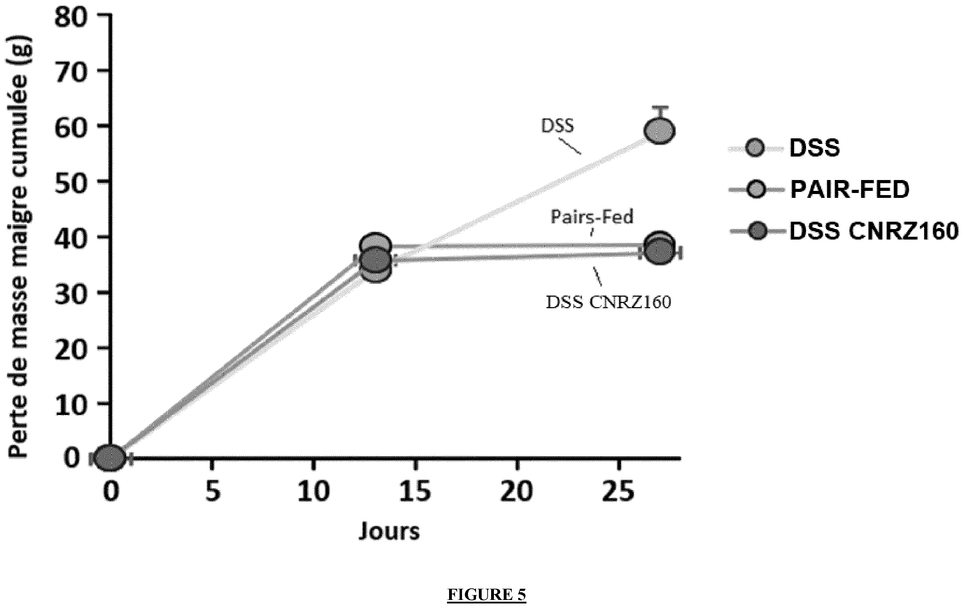

La Figure 5 illustre la masse maigre cumulée perdue par différents groupes de rats dans les jours post-administration de DSS.

Les groupes sont les suivants : DSS : groupe de rats traités au DSS (contrôle enflammé) ; Pair Fed = animaux pair-fed par rapport au groupe DSS ; DSS CNRZ160 : animaux traités au DSS et supplémentés en *Streptococcus thermophilus* CNRZ160.

Abscisse : nombre de jours post-injection de DSS.

Ordonnée : Perte de masse maigre cumulée (g).

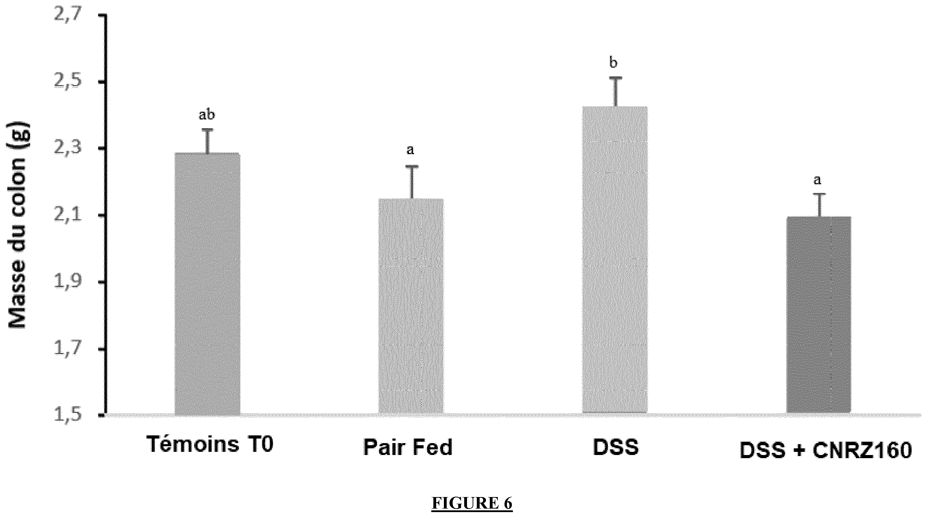

La Figure 6 illustre la masse du colon de différents groupes de rats après 28 jours d'administration de DSS.

Les groupes sont les suivants, de gauche à droite : Témoins T0 : groupe d'animaux euthanasiés au début de l'expérimentation pour mesurer le poids des muscles des rats avant traitement au DSS ; Pair Fed = animaux pair-fed par rapport au groupe DSS ; DSS : groupe de rats traités au DSS (contrôle enflammé) ; DSS + CNRZ160 : animaux traités au DSS et supplémentés en *Streptococcus thermophilus* CNRZ160.

Chaque barre présentant une lettre différente d'une autre est significativement différente. Ainsi, les barres a sont statistiquement différentes de la barre b. La barre ab n'est pas statistiquement différentes des barres a et b.

Abscisse : groupes d'animaux testés.

Ordonnée : Masse du colon (g).

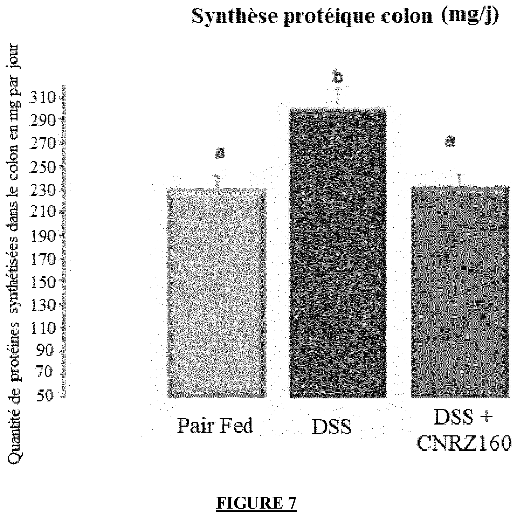

La Figure 7 illustre la synthèse cumulée de protéines du colon en mg par jour de différents groupes de rats (de gauche à droite) : Pair Fed = animaux pair-fed par rapport au groupe DSS ; DSS = groupe d'animaux traités au Dextran Sulfate Sodium (DSS) pendant 28 jours ; DSS + CNRZ160 = animaux traités au DSS pendant 28 jours et supplémentés en *Streptococcus thermophilus* CNRZ160.

Chaque barre présentant une lettre différente d'une autre est significativement différente. Ainsi, les barres a sont statistiquement différentes de la barre b.

Abscisse : groupes d'animaux testés.

Ordonnée : Quantité de protéines synthétisées dans le colon par jour (mg/jour).

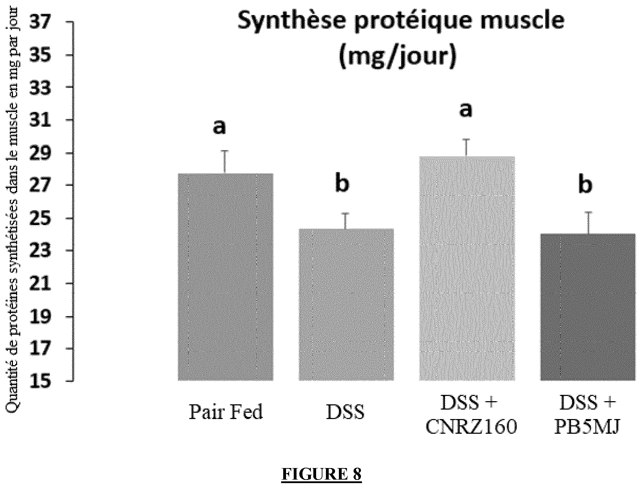

La Figure 8 illustre la synthèse cumulée de protéines du muscle gastrocnémien en mg par jour de différents groupes de rats (de gauche à droite) : Pair Fed = animaux pair-fed par rapport au groupe DSS ; DSS = groupe d'animaux traités au Dextran Sulfate Sodium (DSS) pendant 28 jours ; DSS + CNRZ160 = animaux traités au DSS pendant 28 jours et supplémentés en *Streptococcus thermophilus* CNRZ160 ; DSS + PB5MJ = animaux traités au DSS pendant 28 jours et supplémentés en *Streptococcus thermophilus* PB5MJ.

Chaque barre présentant une lettre différente d'une autre est significativement différente. Ainsi, les barres a sont statistiquement différentes des barres b.

Abscisse : groupes d'animaux testés.

Ordonnée : Quantité de protéines synthétisées dans le muscle par jour (mg/jour).

### Description détaillée de l'invention

Les présents inventeurs ont conduit des travaux approfondis afin d'identifier la capacité d'une souche spécifique de *Streptococcus thermophilus* de référence CNRZ160 à traiter et/ou prévenir une affection intestinale inflammatoire et/ou un trouble provoqué par cette affection intestinale inflammatoire, chez un individu, en particulier les troubles choisis dans la liste constituée d'une perte de poids, d'une perte de masse maigre, d'une perte musculaire, d'une hypertrophie du côlon, d'une altération de la perméabilité intestinale et de l'une quelconque des combinaisons de ces troubles, et notamment la perte de masse en muscles striés.

L'amyotrophie, également appelée myatrophie ou atrophie musculaire, correspond à une diminution du nombre total de fibres musculaires, et plus particulièrement des fibres de type II, chez un individu, et ainsi à un affaiblissement de cet individu.

Cette fonte musculaire peut avoir une évolution plus ou moins rapide dans le temps, être localisée ou générale, et avoir diverses origines.

Ainsi, une atrophie musculaire est observée chez des individus dont l'organisme est partiellement ou complètement immobilisé, que ce soit suite à la pose d'un plâtre lors d'une fracture ou lors d'un alitement prolongé. Cette fonte musculaire tient principalement à un manque d'activité musculaire et reste bénigne et réversible chez l'individu jeune et sans pathologie chronique.

Dans certains cas, cette amyotrophie est liée à une myopathie d'origine héréditaire, parmi lesquelles peuvent être citées la myopathie de Duchenne, la myopathie facio-scapulo-humérale ou la maladie de Steiner.

Il peut également s'agir d'une amyotrophie acquise, résultant soit d'une inflammation, parmi lesquelles par exemple une inflammatoire intestinale et/ou une inflammation des muscles (et l'on parle alors de polymyosite), soit d'un traitement ayant pour effet secondaire une telle perte musculaire. Il a ainsi été observé qu'une administration à haute dose et sur le long terme de cortisone peut être à l'origine d'une telle amyotrophie.

Certaines amyotrophies ont une origine neurologique, comme c'est par exemple le cas dans la maladie de Charcot, de l'amyotrophie spinale, de la poliomyélite ou de lésions nerveuses.

Elle peut enfin être liée à l'âge. En effet, la dystrophie musculaire liée à l'âge, également appelé sarcopénie, est une pathologie définie de manière consensuelle par le groupe européen EWGSOP (le European Working Group on Sarcopenia in Older People - Cruz-Jentoft AJ et al. ; Age Ageing, 2010 Jul; 39(4) : 412-423), et résulte en une perte progressive et élevée de la masse, de la force et de la fonction musculaire au cours du vieillissement. Cette dégénérescence commence dès l'âge de 30 ans et peut représenter une perte progressive de l'ordre de 3 à 8% de la masse musculaire par décennie, avec une accélération de cette dégénérescence dès 50 ans. Cette baisse de masse musculaire est par ailleurs concomitante à une augmentation de la masse grasse.

Dans tous les cas, cette perte de masse musculaire peut conduire à une perte d'autonomie partielle ou totale des individus concernés, résultant en des difficultés à réaliser les activités de la vie quotidienne, accompagnés éventuellement de troubles du maintien de la posture, troubles de la marche, troubles de l'équilibre, une diminution de la masse osseuse, une plus grande fatigabilité, des troubles des capacités cardio-vasculaires et un risque accrue de chutes et donc de fractures, pouvant alors conduire à une immobilisation forcée qui aggraverait encore la situation.

### Souche de Streptococcus thermophilus CNRZ160 de l'invention

Cette souche a été déposée auprès de la CNCM sous le numéro d'accession CNCM I-5334 le 4 juillet 2018.

*Streptococcus thermophilus* est une bactérie lactique, alimentaire et thermophile, présente seulement dans la fermentation du lait où elle est responsable de l'acidification du lait lors de la fabrication des yaourts. Cette bactérie est également présente dans les fromages à pâte pressée cuite à température élevée.

Il a été démontré par le passé que l'administration de bactéries spécifiques, parmi lesquelles *Streptococcus thermophilus* de référence FP4, résultait en une diminution des baisses de performance et de la tension musculaire dans les jours suivant la pratique d'exercices physiques occasionnant des dommages aux muscles (voir JÄGER Ralf et al. ; Nutrients 2016, 8, 642).

AU2015100928 décrit par ailleurs une combinaison probiotique comprenant au moins 2 bactéries choisies dans une liste de 19 membres parmi lesquels *Streptococcus thermophilus* est mentionnée de façon générale, pour traiter ou prévenir la fatigue, promouvoir la synthèse d'ATP, stimuler/augmenter les niveaux d'énergie, traiter ou prévenir les douleurs musculaires, la fatigue musculaire et/ou la dégénération musculaire ou pour promouvoir la réparation musculaire. Il ne s'agit toutefois que d'allégation, la partie expérimentale de cette demande ne comprenant aucun élément supportant de telles propriétés pour cette combinaison de probiotiques. Par ailleurs, comme cela est démontré dans les exemples, les inventeurs ont présentement prouvé que les propriétés particulièrement considérées pour la présente invention ne sont pas possédées par toutes les bactéries du genre *S. thermophilus.*

Ainsi, les inventeurs ont démontré que les propriétés mentionnées ci-dessus de la souche *S. thermophilus* CNRZ160 ne peuvent pas être attribuées à l'espèce *S*. *thermophilus*, étant donné que l'existence de cette propriété est imprévisible pour une souche donnée de *S. thermophilus.* En effet, ces activités sont illustrées dans les exemples comme indiqué ci-dessus, dans lesquels un essai comparatif a été conduit avec une souche de *S*. *thermophilus* ne faisant pas partie de l'invention, à savoir la souche de référence PB5MJ, qui ne possède pas les propriétés de la souche CNRZ160.

Une dose journalière adaptée d'une souche bactérienne selon l'invention est de 10⁷ à 10¹¹ unités formant des colonies (ufc) en tant que médicament, par exemple sous la forme d'une dose journalière équivalente à 10⁹ ufc.

Une bactérie selon l'invention est une bactérie probiotique. Une bactérie probiotique selon l'invention désigne un micro-organisme qui, lorsqu'il est ingéré, exerce des effets bénéfiques sur la santé humaine. Celui-ci peut avantageusement être ingéré sous une forme vivante.

La souche bactérienne de l'invention peut être administrée à un individu de différentes façons, à savoir par voie orale ou rectale. Une bactérie selon l'invention est, de préférence, administrée par la voie orale.

Selon un mode de réalisation préféré, la souche bactérienne de l'invention est comprise dans une composition comprenant un milieu physiologiquement acceptable. Une telle composition est, de préférence, pour une administration par voie orale, et en particulier sous la forme d'un supplément alimentaire.

### Compositions

La présente invention concerne en outre une composition comprenant, dans un milieu physiologiquement acceptable, au moins la souche bactérienne *Streptococcus thermophilus* de référence CNRZ160.

Une composition de l'invention est, de préférence, une composition orale ou rectale, plus préférablement une composition orale.

Selon un mode de réalisation, une composition de l'invention est une composition orale, c'est-à-dire qu'elle est prévue pour une administration orale à un sujet.

Une telle composition peut être sous la forme d'une suspension, d'un comprimé, d'une pilule, d'une capsule, d'un granulé ou d'une poudre.

La composition selon l'invention pour une administration par voie orale peut être choisie dans le groupe constitué d'un produit alimentaire, d'une boisson, d'un produit pharmaceutique, d'un nutraceutique, d'un additif alimentaire, d'un supplément alimentaire ou d'un produit laitier, et est, en particulier, un produit laitier ou un supplément alimentaire.

Selon un mode de réalisation préféré, une composition selon l'invention est un produit laitier.

Un produit laitier pour une administration orale selon l'invention peut être choisi dans la liste constituée d'un yaourt, d'un lactosérum, de beurre, de crème, de lait entier, de lait partiellement écrémé, de lait totalement écrémé, d'un fromage, en particulier un fromage à pâte cuite, en particulier les fromages à pâte cuite à une température supérieure ou égale à 45°C, tels que par exemple l'emmental, le comté ou le parmesan.

Selon un mode de réalisation préféré, une composition selon l'invention est un supplément alimentaire.

Un supplément alimentaire pour une administration orale peut être présent dans des capsules, des gélules, des capsules molles, des comprimés, des comprimés dragéifiés, des pilules, des pâtes, des pastilles, des gommes, des solutions ou émulsions buvables, un sirop ou un gel.

Avantageusement, une composition selon l'invention, prévue pour une administration orale, peut être pourvue d'un enrobage résistant au suc gastrique, afin d'assurer que la souche bactérienne de l'invention comprise dans ladite composition puisse traverser l'estomac sans être endommagée. La libération de la souche bactérienne peut ainsi se produire pour la première fois dans le tractus intestinal supérieur.

Un supplément alimentaire selon l'invention peut comprendre en outre un édulcorant, un stabilisant, un antioxydant, un additif, un agent aromatisant et/ou un colorant.

La formulation de celui-ci est effectuée au moyen des procédés usuels pour produire des comprimés dragéifiés, des gélules, des gels, des hydrogels pour libération contrôlée, des émulsions, des comprimés ou des capsules.

Dans un autre mode de réalisation de l'invention, une composition contenant la souche bactérienne de l'invention est administrée par voie intrarectale.

De préférence, une administration rectale est conduite sous la forme d'un suppositoire, un lavement ou une mousse.

En particulier, une composition de l'invention est adaptée pour l'administration d'une dose journalière représentant de 10⁷ à 10¹¹ unités formant des colonies (ufc) en tant que médicament, de préférence une dose journalière équivalente à 10⁹ ufc.

Par exemple, une composition selon l'invention peut être administrée à un individu en ayant besoin à une dose journalière unique de 1 g contenant la souche bactérienne *S. thermophilus* CNRZ160 de l'invention en une quantité équivalente à une dose comprise entre 10⁷ et 10¹¹ ufc, de préférence 10⁹ ufc.

Dans un autre exemple, une composition selon l'invention peut être administrée à un individu en ayant besoin à une dose journalière unique de 0,2 g contenant la souche bactérienne *S. thermophilus* CNRZ160 de l'invention en une quantité équivalente à une quantité comprise entre 10⁷ et 10¹¹ ufc, de préférence 10⁹ ufc.

Dans un autre exemple, une composition selon l'invention peut être administrée à un individu en ayant besoin deux fois par jour sur la base de deux doses de 1 g, chaque dose contenant, indépendamment, la souche bactérienne *S. thermophilus* CNRZ160 de l'invention en une quantité équivalente à une quantité comprise entre 5.10⁶ et 5.10¹⁰ ufc (sur la base du poids sec), de préférence 5.10⁸ ufc, de sorte que la dose journalière totale de souche bactérienne *S. thermophilus* CNRZ160 de l'invention administrée à l'individu soit telle qu'indiquée ci-dessus.

Une composition selon l'invention peut comprendre en outre au moins l'un parmi : des antioxydants, des huiles de poisson, DHA, EPA, des vitamines, des minéraux, des phytonutriments, une protéine, un lipide, des probiotiques et des combinaisons de ceuxci.

L'invention est décrite ci-dessous de façon plus détaillée au moyen des exemples suivants qui sont présentés à titre d'illustration uniquement.

Toutes les références à des pourcentages sont des pourcentages en poids sauf indication contraire.

### EXEMPLES

### Exemple I

### A. Induction de colite par DNBS et administration de bactéries

La souche de *Streptococcus thermophilus* CNRZ160 selon l'invention a dans un premier temps été testée, et comparée à une autre souche de *S. thermophilus*, PB5MJ, pour sa capacité à limiter *in vivo* une inflammation intestinale chez un individu.

Une inflammation bas grade est induite chez des souris par injection d'une faible dose de DNBS.

Le protocole de colite induite par DNBS est réalisé comme précédemment décrit (Martín R, et al. Inflamm Bowel Dis. Mars 2014 ; 20(3):417-30).

Brièvement, les souris sont anesthésiées avec de l'isoflurane (Abbott, Abbott Park, IL) et un segment de 10 cm de longueur de tubulure PE-90 (ClayAdam, Parsippany, NJ) est fixé à une seringue de tuberculine et inséré à 3,5 cm dans le côlon.

La colite est induite par injection intrarectale (i.r.) par l'intermédiaire de ce tube de 200 mg/kg de solution de DiNitroBenzène-acide Sulfonique (DNBS) (ICN, Biomedical Inc.) dans de l'éthanol à 30 % (EtOH).

Les souris témoins (sans colite) reçoivent uniquement de l'éthanol (EtOH).

Les souris sont alimentées avec 6 % saccharose dans de l'eau de boisson pendant 3 premiers jours après l'injection de DNBS pour prévenir la déshydratation (période DNBS). 10 jours après la période DNBS, 200 µl contenant 1×10⁹ UFC d'une des souches bactériennes discutées ci-après sont administrées par voie intragastrique, chaque jour pendant 10 jours (période de gavage).

La colite est réactivée 21 jours après la première injection de DNBS (période de récupération) avec une deuxième injection de 100 mg/kg de solution de DNBS.

Les groupes d'étude sont comme suit : groupe témoin non-colite (Ethanol + PBS), groupe témoin colite (DNBS + PBS), groupe de souche de *S. thermophilus* PB5MJ (DNBS + PB5MJ) et *S. thermophilus* CNRZ160 (DNBS + CNRZ160).

### B. Mesure de la perméabilité intestinale comme marqueur de l'inflammation

Des souris (n=8/groupe) ont été gavées, ou non, sur la base du protocole indiqué ci-dessus avec les souches de *S. thermophilus* PB5MJ ou CNRZ160, poussées dans du lait.

Une augmentation de la perméabilité intestinale est ensuite observée chez ces souris par un dosage de FITC dans le sang.

On observe en effet un doublement de la concentration de FITC entre les groupes PBS éthanol (contrôle non enflammé) et PBS DNBS (contrôle enflammé) ce qui traduit une augmentation de la perméabilité.

Les résultats obtenus sont représentés en Figure 1.

Le gavage par la souche CNRZ160 permet un rétablissement complet de la perméabilité intestinale.

A l'inverse, le gavage par la souche PB5MJ n'a pas d'effet sur la restauration de la perméabilité intestinale.

Ainsi, une souche de référence CNRZ160 selon l'invention présente effectivement un effet anti-inflammatoire.

### Exemple II - Mesure de l'effet de la souche CNRZ160 sur la perte de poids

Une inflammation aigue est induite chez des souris par injection d'une forte dose de DNBS.

Le protocole de colite induite par DNBS est réalisé comme précédemment décrit (Martín R, *et al.* FEMS Microbiology review, 2017, S49-S70)).

Brièvement, les souris sont anesthésiées avec de l'isoflurane (Virbac France Espace Azur Mercantour - 3e rue - LID - 06510 Carros) et un segment de 10 cm de longueur de tubulure PE-90 (ClayAdam, Parsippany, NJ) est fixé à une seringue de tuberculine et inséré à 3,5 cm dans le côlon.

La colite est induite par injection intrarectale (i.r.) par l'intermédiaire de ce tube de 3000 mg/kg de solution de DiNitroBenzène-acide Sulfonique (DNBS) (ICN, Biomedical Inc.) dans de l'éthanol à 30 % (EtOH).

Les souris témoins (sans colite) reçoivent uniquement de l'éthanol (EtOH).

Six jours avant la période DNBS, 200 µl contenant 1×10⁹ UFC d'une des souches bactériennes discutées ci-après sont administrées par voie intragastrique, chaque jour pendant 10 jours (période de gavage). Le DNBS est administré à t0 et les souris sont sacrifiées 4 jours après l'injection de DNBS.

Des souris DNBS ont été préparées et gavées avec la souche de référence CNRZ160 poussée dans du lait.

Le poids de ces souris (n=8/groupe) est ensuite mesuré tous les jours pendant 4 jours.

Les résultats obtenus sont représentés en Figure 2.

Les souris Lait-DNBS (contrôle inflammé) ont perdu jusqu'à 10% de leur poids après 2 jours (D2) pour récupérer ensuite un poids normal correspondant à celui du groupe PBS (contrôle non-enflammé).

Les souris gavées avec la CNRZ160 perdent moins de poids que le groupe contrôle inflammé. La différence est significative à D2 par rapport au groupe Lait-DNBS.

### Exemple III

La souche de *Streptococcus thermophilus* CNRZ160 selon l'invention a également été testée, et comparée à une autre souche de *S. thermophilus*, PB5MJ, dans un autre modèle d'inflammation intestinale, pour sa capacité à :
- limiter *in vivo* la fonte musculaire d'animaux souffrant d'une inflammation intestinale.
- limiter la mort de tels individus.
- limiter la perte de masse maigre.
- limiter l'hypertrophie du colon.

Les différents protocoles et résultats obtenus en ce sens sont présentés ci-après.

### A. Induction de colite par DSS et administration de bactéries

Pour induire la colite, de l'eau buvable supplémentée avec 2% ((w/v) de dextrane sulfate sodium (DSS; MP Biomedicals, LLC, Aurora, OH, USA) est administrée aux souris pendant 7 jours. Les souris récupèrent par la suite pendant 5 jours en buvant de l'eau non supplémentée (Figure 7a). Du 6- formylindolo[3,2-b]carbazole (Ficz) obtenu d'Enzo Life Sciences (Lausanne, Suisse), et resuspendu dans du dimethyl sulfoxide (DMSO; Sigma-Aldrich), est administré en intrapéritonéal 1 jour après l'administration de DSS (1 µg/souris).

Les contrôles consistent en des souris injectées avec du DMSO seul.

### B. Essais sur la perte de masse musculaire

**a.** Comme indiqué ci-dessus, les inventeurs ont étudié l'impact de la prise de *S. thermophilus* CNRZ160 sur la fonte musculaire au cours d'une inflammation intestinale générée chimiquement par l'ingestion de Dextran Sulfate Sodium (4% DSS dans l'eau de boisson) sur une période de 28 jours.

Les groupes expérimentaux comparés sont les suivants :
- un groupe d'animaux témoins euthanasiés au début de l'expérimentation pour mesurer le poids des muscles avant traitement au DSS (groupe Témoins T0) ;
- un groupe témoins d'animaux traités au DSS sans administration de S. *thermophilus* selon l'invention (groupe DSS) ;
- un groupe d'animaux traités au DSS et supplémentés en *Streptococcus thermophilus* CNRZ160, appelé groupe « DSS + CNRZ160 » ; et
- un groupe comparatif d'animaux traités au DSS et supplémentés en *Streptococcus thermophilus* PB5MJ, appelé groupe « DSS + PB2 ».
- un groupe d'animaux pair-fed du groupe DSS, dit groupe PF ou groupe Pair Fed, dans la mesure où l'ingestion de DSS se traduit par une diminution légère de l'ingéré chez les animaux ce qui a un impact *per se* sur la masse musculaire. Ainsi, les animaux du groupe PF ne sont pas traités au DSS mais ne reçoivent quotidiennement qu'une quantité de nourriture égale à celle ingérée par les animaux de tous les groupes DSS avec ou sans les souches bactériennes.

Les quantités d'aliments ingérés par les animaux des différents groupes DSS, PF, DSS + CNRZ160 et DSS + PB2 sont identiques.

Les effets des traitements expérimentaux sur le poids des animaux est par ailleurs contrôlé à l'aide d'une balance.

### b. Résultats

Le pair-feeding, correspondant à un ingéré inférieur à l'*ad libitum,* se traduit par une perte de poids des animaux, comme illustré en Figure 3. Cette perte de poids est accentuée avec l'ingestion du DSS.

L'effet des divers traitements expérimentaux sur le poids des muscles des animaux, et en particulier sur le poids du muscle gastrocnémien, est par ailleurs également représenté en Figure 3.

Il peut ainsi être observé qu'à la fin des 28 jours de traitement au DSS, les animaux supplémentés en *S. thermophilus* CNRZ160 (groupe DSS + CNRZ160) présentent une masse musculaire statistiquement supérieure à celle du groupe d'animaux DSS, ce qui n'est pas le cas pour le groupe d'animaux supplémentés en *S. thermophilus* PB5MJ (groupe DSS + PB2).

La masse des animaux du groupe DSS + CNRZ160 est statistiquement similaire à celle des animaux du groupe PF.

Il est ainsi démontré que la supplémentation des animaux avec la bactérie *S. thermophilus* CNRZ160 permet de limiter la fonte musculaire des animaux au cours de la période expérimentale de traitement au DSS, et que toutes les souches de *S. thermophilus* ne possèdent pas de telles propriétés, comme démontré avec la souche PB5MJ.

### C. Essais sur la mortalité

**a.** Les souris mentionnées ci-dessus traitées au DSS ont, préalablement à l'induction de la colite, été gavées pendant 5 jours avec la souche CNRZ160 ou la souche PB5MJ.

Une courbe de survie a été mesurée pour chacune des différentes populations de souris : un groupe de souris (n=8) traitées au DSS mais n'ayant préalablement été traités qu'au PBS (contrôle - PBS DSS), un groupe de souris (n=8) traitées au DSS et préalablement gavées avec du CNRZ160 (CNRZ160 DSS) et un groupe de souris (n=8) traitées au DSS et préalablement gavées avec du PB5MJ (PB5MJ DSS).

### b. Résultats

Les résultats obtenus sont représentés Figure 4.

Le traitement au DSS entraine la mort de 25% des souris dans le groupe contrôle non-traité (PBS DSS).

On observe une mortalité plus importante dans le groupe pré-gavé avec la PB5MJ, à savoir de l'ordre de 60%.

A l'inverse, aucune mortalité n'est observée dans le groupe de souris pré-gavée avec la CNRZ 160.

### D. Essais sur la perte de masse maigre

**a.** Des rats telles que mentionnés ci-dessus traitées au DSS ont, préalablement à l'induction de la colite, été gavées avec la souche CNRZ160.

Une courbe représentant la perte de masse maigre cumulée a été réalisée pour chacune des différentes populations de rats testées, à savoir :
- un groupe de rats (n=12) traitées au DSS mais n'ayant préalablement été traités qu'au PBS (contrôle - DSS),
- un groupe de de rats (n=12) traitées au DSS et préalablement gavées avec du CNRZ160 (DSS CNRZ160), et
- un groupe de de rats (n=12) pair-fed du groupe DSS, non traités au DSS mais ne recevant quotidiennement qu'une quantité de nourriture égale à celle ingérée par les animaux du groupe DSS.

La masse maigre est mesurée à l'aide de l'ÉchoMRI-700.

L'ÉchoMRI-700 est un système de résonance magnétique nucléaire quantitatif qui permet d'obtenir des mesures précises des paramètres de composition corporelle: masse corporelle grasse totale, masse corporelle maigre totale, eau libre et eau totale corporelle chez le rat ou la souris.

La prise de mesures ne nécessite pas d'anesthésie ou de sédation.

La mise au point et la calibration de l'équipement sont automatiques.

Les radiations sont non-ionisantes et très reproductibles.

### b. Résultats

Les résultats obtenus sont représentés Figure 5.

La perte de masse maigre a été mesurée sur 25 jours à compter du traitement au DSS.

Une diminution constante de la masse maigre est observée pendant toute la durée des mesures, les animaux du groupe DSS perdant environ 60 g de masse maigre au bout des 25 jours d'observations.

A l'inverse, la perte de masse maigre observée dans le groupe traité avec la souche CNRZ160 est identique à celle observée dans la population Pair-Fed, à savoir environ 40g au bout de 25 jours d'observations, ce qui est bien inférieur au 60g de pertes observées dans le groupe DSS. On observe plus particulièrement un arrêt, ou tout du moins un très fort ralentissement de la perte de masse maigre dès 12 jours dans les groupes Pair-Fed et DSS CNRZ160.

### E. Essais sur la limitation de l'hypertrophie du colon

**a.** Des rats tels que mentionnés ci-dessus traitées au DSS ont, préalablement à l'induction de la colite, été gavées avec la souche CNRZ160.

Le colon de chaque animal a été isolé et précautionneusement retiré et pesé pour en évaluer la masse.

Plus particulièrement, la masse du colon de différentes populations de rats a été mesurée après 28 jours d'administration de DSS :
- un groupe d'animaux témoins (n=12) euthanasiés au début de l'expérimentation pour mesurer le poids du colon avant traitement au DSS (groupe Témoins T0) ;
- un groupe de rats (n=12) pair-fed du groupe DSS, non traités au DSS mais ne recevant quotidiennement qu'une quantité de nourriture égale à celle ingérée par les animaux du groupe DSS,
- un groupe de rats (n=12) traités au DSS mais n'ayant préalablement été traités qu'au PBS (contrôle - DSS), et
- un groupe de rats (n=12) traités au DSS et préalablement gavées avec du CNRZ160 (DSS CNRZ160).

### b. Résultats

Les résultats obtenus sont représentés Figure 6.

Une hypertrophie du colon est observée dans le groupe DSS, avec une augmentation significative de la masse du colon dans cette population d'individus comparativement aux groupes Témoins T0.

A l'inverse, une légère diminution de la masse du colon est observée dans la population Pair Fed.

De même, la population d'individus traités à la fois au DSS et avec la souche CNRZ160 présentent un colon de masse similaire à celle du groupe Pair Fed. Ainsi, il apparait que le traitement à l'aide de la souche CNRZ160 permet avantageusement de réduire les effets du DSS induisant l'hypertrophie du colon quand celle-ci est coadministrée avec le DSS pendant 28 jours.

### F. Essais sur la synthèse protéique du colon et du muscle gastrocnémien

**a.** La synthèse protéique du colon ou du muscle gastrocnémien de différentes populations de rats a été mesurée après 28 jours d'administration de DSS.

Plus particulièrement, la synthèse protéique au niveau du colon les différents groupes de population suivants ont été mesurés après 28 jours d'administration de DSS :
- un groupe de rats *(n=12)* pair-fed du groupe DSS, non traités au DSS mais ne recevant quotidiennement qu'une quantité de nourriture égale à celle ingérée par les animaux du groupe DSS,
- un groupe de rats (n=12) traités au DSS mais n'ayant été traités qu'au PBS (contrôle - DSS), et
- un groupe de rats (n=12) traités au DSS et traité simultanément avec la bactérie CNRZ160 (DSS CNRZ160).

Concernant, la synthèse protéique au niveau du muscle gastrocnémien, les différents groupes de population suivants ont été mesurés après 28 jours d'administration de DSS :
- un groupe de rats (n=12) pair-fed du groupe DSS, non traités au DSS mais ne recevant quotidiennement qu'une quantité de nourriture égale à celle ingérée par les animaux du groupe DSS,
- un groupe de rats (n=12) traités au DSS mais n'ayant été traités qu'au PBS (contrôle - DSS),
- un groupe de rats (n=12) traités au DSS et traités simultanément avec la bactérie CNRZ160 (DSS + CNRZ160), et
- un groupe de rats (n=12) traités au DSS et traités simultanément avec la bactérie PB5MJ (DSS + PB5MJ).

Pour la mesure de la synthèse protéique, la technique de large dose est utilisée.

150 µmoles/100g (100% Valine 1-¹³C) sont injectées en intraveineux 20 minutes avant le sacrifice de l'animal.

La mesure des enrichissements (Valine 1-¹³C) plasmatiques, intra-tissulaires et intra-protéines du muscle gastrocnémien ou du colon permet de déterminer une vitesse d'incorporation des acides aminés dans les protéines et donc de donner une valeur de synthèse protéique.

Les détails de la procédure de préparation des échantillons, de la mesure des enrichissements en 13C valine et du calcul sont détaillés dans Jarzaguet et al. 2018 (Food Funct. 2018 Dec 13 ;9(12) :6526-6534).

### b. Résultats

Les résultats obtenus pour la synthèse protéique au niveau du colon sont représentés en Figure 7. Les résultats obtenus pour la synthèse protéique au niveau du muscle gastrocnémien sont représentés en Figure 8.

Ainsi, au niveau du colon tout d'abord, on observe un retour à la normale de la synthèse protéique du colon lors d'un traitement avec la souche CNRZ160.

Similairement, on observe un maintien de la synthèse protéique au niveau du muscle gastrocnémien dans les populations traitées avec la souche CNRZ160. Cela n'est toutefois pas le cas chez les individus traités avec la souche PB5MJ.

### G. Analyse statistique

L'analyse statistique est effectuée au moyen du logiciel Sigma Plot SigmaPlot 12, Systat software, San Jose, USA). Toutes les données sont exprimées sous forme de moyenne +/- SEM.

Les comparaisons sont réalisées suivant les cas par ANOVA 1 facteur suivi d'un test post hoc LSD Fisher. Une valeur p inférieure à 0,05 est considérée comme étant significative.

## Revendications

1. Souche bactérienne de l'espèce *Streptococcus thermophilus* déposée auprès de la CNCM sous le numéro d'accession CNCM I-5334, pour son utilisation dans la prévention et/ou le traitement d'une affection intestinale inflammatoire et/ou d'un trouble provoqué par ladite affection intestinale inflammatoire, chez un individu, ledit trouble provoqué par ladite affection intestinale inflammatoire étant choisi dans la liste constituée d'une perte de poids, d'une perte de masse maigre, d'une perte musculaire, d'une hypertrophie du côlon, d'une altération de la perméabilité intestinale et de l'une quelconque des combinaisons de ces troubles.

2. Souche bactérienne pour son utilisation selon la revendication 1, **caractérisée en ce qu'**elle est mise en œuvre sous une forme vivante, semi-active, inactivée ou morte.

3. Souche bactérienne pour son utilisation selon la revendication 1 ou 2, **caractérisée en ce que** l'individu est un mammifère, en particulier un humain.

4. Souche bactérienne pour son utilisation selon l'une quelconque des revendications 1 à 3, ledit trouble provoqué par cette affection intestinale inflammatoire étant la perte de masse maigre et musculaire.

5. Souche bactérienne pour son utilisation selon l'une quelconque des revendications 1 à 4, **caractérisée en ce que** l'affection intestinale inflammatoire est une maladie inflammatoire chronique de l'intestin, et est en particulier choisie dans le groupe constitué de la maladie de Crohn, la recto-colite hémorragique et la pochite, en particulier la maladie de Crohn et la recto-colite hémorragique.

6. Souche bactérienne pour son utilisation selon l'une quelconque des revendications 1 à 5, **caractérisée en ce que** la souche bactérienne est comprise dans une composition comprenant un milieu physiologiquement acceptable, de préférence dans une composition orale.

7. Souche bactérienne pour son utilisation selon l'une quelconque des revendications 1 à 6, **caractérisée en ce que** la composition est adaptée pour l'administration d'une dose journalière représentant de 10⁷ à 10¹¹ unités formant des colonies (ufc), notamment en tant que médicament, de préférence sous la forme d'une dose journalière équivalente à 10⁹ ufc.

8. Souche bactérienne pour son utilisation selon la revendication 7, **caractérisée en ce que** la composition est pour une administration par voie orale et est choisie dans le groupe constitué d'un produit alimentaire, d'une boisson, d'un produit pharmaceutique, d'un nutraceutique, d'un additif alimentaire, d'un supplément alimentaire et d'un produit laitier, et est en particulier un produit laitier ou un supplément alimentaire.

## Patentansprüche

1. Bakterienstamm der Spezies *Streptococcus thermophilus*, hinterlegt bei der CNCM unter der Zugangsnummer CNCM I-5334, für die Verwendung bei der Verhinderung und/oder Behandlung einer entzündlichen Darmerkrankung und/oder einer Störung, die durch die entzündliche Darmerkrankung verursacht wird, in einem Individuum, wobei die Störung, die durch die entzündliche Darmerkrankung verursacht wird, ausgewählt ist aus der Liste, bestehend aus Gewichtsverlust, Verlust an magerer Körpermasse, Muskelabbau, Hypertrophie des Kolons, Änderung der Darmpermeabilität und jedweder Kombination dieser Störungen.

2. Bakterienstamm für die Verwendung nach Anspruch 1, **dadurch gekennzeichnet, dass** er in lebender, semiaktiver, inaktivierter oder toter Form bereitgestellt wird.

3. Bakterienstamm für die Verwendung nach Anspruch 1 oder 2, **dadurch gekennzeichnet, dass** das Individuum ein Säugetier, insbesondere ein Mensch ist.

4. Bakterienstamm für die Verwendung nach einem der Ansprüche 1 bis 3, wobei es sich bei der Störung, die durch diese entzündliche Darmerkrankung verursacht wird, um den Verlust an magerer Körpermasse und Muskelabbau handelt.

5. Bakterienstamm für die Verwendung nach einem der Ansprüche 1 bis 4, **dadurch gekennzeichnet, dass** die entzündliche Darmerkrankung eine chronische Entzündungskrankheit des Darms ist und insbesondere ausgewählt ist aus der Gruppe, bestehend aus Morbus Crohn, Colitis ulcerosa und Pouchitis, insbesondere Morbus Crohn und Colitis ulcerosa.

6. Bakterienstamm für die Verwendung nach einem der Ansprüche 1 bis 5, **dadurch gekennzeichnet, dass** der Bakterienstamm in einer Zusammensetzung, die ein physiologisch akzeptables Medium umfasst, vorzugsweise in einer oralen Zusammensetzung, enthalten ist.

7. Bakterienstamm für die Verwendung nach einem der Ansprüche 1 bis 6, **dadurch gekennzeichnet, dass** die Zusammensetzung zur Verabreichung einer Tagesdosis von 10⁷ bis 10¹¹ koloniebildenden Einheiten (cfu), insbesondere als Medikament, vorzugsweise in Form einer Tagesdosis, die 10⁹ cfu entspricht, ausgelegt ist.

8. Bakterienstamm für die Verwendung nach Anspruch 7, **dadurch gekennzeichnet, dass** die Zusammensetzung zur oralen Verabreichung bestimmt ist und ausgewählt ist aus der Gruppe bestehend aus einem Lebensmittelprodukt, einem Getränk, einem pharmazeutischen Produkt, einem Nutrazeutikum, einem Lebensmittelzusatzstoff, einem Nahrungsergänzungsmittel und einem Milchprodukt, und insbesondere ein Milchprodukt oder ein Nahrungsergänzungsmittel ist.

## Claims

1. Bacterial strain of the species *Streptococcus thermophilus* deposited with the CNCM under accession number CNCM 1-5334, for use thereof in the prevention and/or treatment of an inflammatory intestinal disorder and/or of a disorder caused by said inflammatory intestinal disorder, in an individual, said disorder caused by said inflammatory intestinal disorder being selected from the list consisting of weight loss, loss of lean mass, muscle loss, hypertrophy of the colon, a change in intestinal permeability and any combination of these disorders.

2. Bacterial strain for use thereof according to Claim 1, **characterized in that** it is employed in a live, semi-active, inactivated or dead form.

3. Bacterial strain for use thereof according to Claim 1 or 2, **characterized in that** the individual is a mammal, in particular a human.

4. Bacterial strain for use thereof according to any one of Claims 1 to 3, said disorder caused by this inflammatory intestinal disorder being loss of lean mass and of muscle mass.

5. Bacterial strain for use thereof according to any one of Claims 1 to 4, **characterized in that** the inflammatory intestinal disorder is a chronic inflammatory disease of the intestine, and is in particular selected from the group consisting of Crohn's disease, ulcerative colitis and pouchitis, in particular Crohn's disease and ulcerative colitis.

6. Bacterial strain for use thereof according to any one of Claims 1 to 5, **characterized in that** the bacterial strain is included in a composition comprising a physiologically acceptable medium, preferably in an oral composition.

7. Bacterial strain for use thereof according to any one of Claims 1 to 6, **characterized in that** the composition is suitable for administration of a daily dose representing from 10⁷ to 10¹¹ colony forming units (CFU), notably as a medicinal product, preferably in the form of a daily dose equivalent to 10⁹ CFU.

8. Bacterial strain for use thereof according to Claim 7, **characterized in that** the composition is for administration by the oral route and is selected from the group consisting of a food product, a drink, a pharmaceutical product, a nutraceutical, a food additive, a food supplement and a milk product, and is in particular a milk product or a food supplement.
